# EUROPEAN PATENT APPLICATION

(11) **EP 2 570 479 A1**
(43) Date of publication of application: **20.03.2013**
(21) Application number: 11780551.5
(22) Date of filing: 25.04.2011
(51) Int. Cl.: C12N 9/06, C12N 15/09, H01M 4/90, H01M 8/16

(54) **RECOMBINANT BILIRUBIN OXIDASE AND PRODUCTION METHOD FOR SAME**

(30) Priority: 13.05.2010 JP 2010111186
(71) Applicant: Sony Corporation, Tokyo 108-0075 (JP)
(72) Inventor: KUMITA, Hideyuki, Tokyo 108-0075 (JP); YAMAGUCHI, Daisuke, Tokyo 108-0075 (JP); TOKITA, Yuichi, Tokyo 108-0075 (JP); FUJITA, Shuji, Tokyo 108-0075 (JP)
(74) Representative: Müller - Hoffmann & Partner
(86) International application number: PCT/JP2011/060572
(87) International publication number: WO 2011/142294

(57) **Abstract**

Disclosed is a method of producing a recombinant bilirubin oxidase, including a step wherein a bilirubin oxidase gene derived from imperfect filamentous fungus Myrothecium verrucaria is introduced to Escherichia coli and expression thereof is allowed. Also disclosed is a recombinant bilirubin oxidase obtained by this production method. This recombinant bilirubin oxidase has an enzymatic activity higher than that of a natural bilirubin oxidase derived from the imperfect filamentous fungus Myrothecium verrucaria.

## Description

### Technical Field

The present invention relates to a recombinant bilirubin oxidase and a method of producing the same. More specifically, the invention relates to a recombinant bilirubin oxidase derived from imperfect filamentous fungus myrothecium verrucaria, and the like.

### Background Art

A "bilirubin oxidase" is an enzyme which catalyzes a reaction of oxidation of bilirubin to biliverdin, and is one kind of enzyme belonging to the multicopper oxidase (the generic term for enzymes having a plurality of copper ions in an active center thereof). Conventionally, the bilirubin oxidase has been widely used as a reagent for clinical tests of liver function and the like (a measurement reagent for bilirubin in blood serum). Besides, in recent years, the bilirubin oxidase has been used also as an electrode enzyme for catalyzing an electrochemical four-electron reduction reaction of oxygen, on the cathode (positive electrode) side of enzyme cells.

As the bilirubin oxidase, in the past, an enzyme sample obtained by extraction from imperfect filamentous fungus Myrothecium verrucaria MT-1 and partial purification of the extract (such a sample will hereinafter be referred to as "natural bilirubin oxidase") has been used (see Patent Document 1). In addition, Non-Patent Document 1 has reported determination of the base sequence of a gene which codes a bilirubin oxidase gene, and construction of an expression system for a recombinant bilirubin oxidase by use of yeast (see Patent Document 2, as well).

### Related Art Documents

### Patent Documents

Patent Document 1: Japanese Patent Laid-open No. Hei 5-199882
Patent Document 2: Japanese Patent Laid-open No. 2005-73660

### Non-Patent Document

Non-Patent Document 1: "Molecular cloning of the gene for bilirubin oxidase from Myrothecium verrucaria and its expression in yeast." J Biol Chem. 1993 Sep 5; 268(25): 18801-9.

### Summary of Invention

### Technical Problems

In order that a bilirubin oxidase derived from imperfect filamentous fungus Myrothecium verrucaria will be supplied in large amounts as a reagent for clinical tests or an electrode enzyme for enzyme cells, construction of an excellent expression system for a recombinant bilirubin oxidase has been desired. However, the conventional methods in which imperfect filamentous fungus Myrothecium verrucaria or yeast is used have had drawbacks in that the expression amount of the enzyme is small or the activity of the enzyme expressed is insufficient.

Accordingly, it is an object of the present invention to provide a simple technique for obtaining a recombinant bilirubin oxidase derived from imperfect filamentous fungus Myrothecium verrucaria with high expression and high activity.

### Technical Solution

In order to solve the above-mentioned problem, the present inventors attempted to cause a bilirubin oxidase gene derived from imperfect filamentous fungus Myrothecium verrucaria, which is a true fungus as a eukaryote, to be expressed in Escherichia coli, which is a bacterium as a prokaryote. As a result, the present inventors found out that using a simple Escherichia coli expression system, it is possible to express and purify a recombinant bilirubin oxidase having sufficient activity. Furthermore, the present inventors made clear that in an Escherichia coli expression system lacking a posttranslational sugar chain modification, unexpectedly, it is possible to obtain a recombinant bilirubin oxidase having an activity higher than that of the natural bilirubin oxidase derived from imperfect filamentous fungus Myrothecium verrucaria.

Based on these findings, according to the present invention, there is provided a recombinant bilirubin oxidase obtained by introducing a bilirubin oxidase gene derived from imperfect filamentous fungus Myrothecium verrucaria to Escherichia coli and allowing expression thereof.

This recombinant bilirubin oxidase has the amino acid sequence of SEQ ID NO: 1, and has a high enzymatic activity higher than that of the natural bilirubin oxidase derived from imperfect filamentous fungus Myrothecium verrucaria. The amino acid sequence of SEQ ID NO: 1 is a sequence obtained by removing 37 residues of a secretory signal peptide from the amino acid sequence of the natural bilirubin oxidase.

In addition, according to the present invention, there is provided a method of producing a recombinant bilirubin oxidase, including a step wherein a bilirubin oxidase gene derived from imperfect filamentous fungus Myrothecium verrucaria is introduced to Escherichia coli and expression thereof is allowed.

In the method of producing a recombinant bilirubin oxidase, it is preferable to use a bilirubin oxidase gene derived from imperfect filamentous fungus Myrothecium verrucaria which has the base sequence of SEQ ID NO: 2, and to use an Escherichia coli strain derived from K-12. The base sequence of SEQ ID NO: 2 is a sequence obtained through removal of a part which codes the secretory signal peptide.

### Advantageous Effect

According to the present invention, there is provided a simple technique for obtaining a recombinant bilirubin oxidase derived from imperfect filamentous fungus Myrothecium verrucaria with high expression and high activity.

### Brief Description of Drawings

FIG. 1 is a schematic diagram illustrating the structure of pET-BO vector.
FIG. 2 is a photograph as a substituent for a drawing, showing the results of SDS-PAGE of a purified recombinant bilirubin oxidase.
FIG. 3 is a spectrogram showing an absorption spectrum of the purified recombinant bilirubin oxidase.
FIG. 4 is a graph showing the evaluation results of activity of the recombinant bilirubin oxidase.

### Mode for Carrying Out the Invention

Now, a preferred mode for carrying out the present invention will be described below referring to the drawings. Incidentally, the mode for carrying out the present invention which will be described below is an example of representative modes for carrying out the invention, and the scope of the invention is not to be thereby construed narrowly.

A recombinant bilirubin oxidase according to the present invention is obtained by introducing a bilirubin oxidase gene derived from imperfect filamentous fungus Myrothecium verrucaria to Escherichia coli and allowing expression thereof. The recombinant bilirubin oxidase is characterized by having an enzymatic activity higher than that of the natural bilirubin oxidase derived from the imperfect filamentous fungus Myrothecium verrucaria (see "Examples"). Incidentally, since the recombinant bilirubin oxidase is obtained by expression in Escherichia coli which has no posttranslational sugar chain modification, the recombinant bilirubin oxidase has no sugar chain modification, unlike the natural bilirubin oxidase and the recombinant (wild-type) bilirubin oxidase obtained by expression in yeast.

As the bilirubin oxidase gene derived from imperfect filamentous fungus Myrothecium verrrucaria, a DNA fragment having the base sequence of SEQ ID NO: 2 can be used. This base sequence is said to be a sequence obtained by removal of a part coding a secretory signal peptide from a DNA which codes the natural bilirubin oxidase. The recombinant bilirubin oxidase obtained by the expression of the bilirubin oxidase gene has the amino acid sequence of SEQ ID NO: 1, which is obtained by removing 37 residues of the secretory signal peptide from the amino acid sequence of the natural bilirubin oxidase. Incidentally, the recombinant bilirubin oxidase according to the present invention may be one that has a sequence obtained by removing methionine corresponding to an initiation codon (the first methionine from the N-terminus) from the amino acid sequence of SEQ ID NO: 1.

As a vector for expression of the recombinant bilirubin oxidase, there can be adopted those plasmid vectors which are commonly used for expression of recombinant proteins. Examples of the vectors include pET22b, pUC18, pHSG398, pHSG399, pRIT2T, pUEX1 to pUEX3, pKK223-3, pINIII 1, pTTQ18, pGEMEX-1, pGH-L9, and pKK233-2.

As a host cell for expression of the recombinant bilirubin oxidase, there can be adopted those Escherichia coli cells which are commonly used for expression of recombinant proteins. As the Escherichi coli, particularly preferable is a strain derived from K-12, examples of which include such strains as BL21 (DE3), BB4, BM25.5, BMH71-18mutS, BW313, C-1a, C600, CJ236, DH1, DH5, DH5α, DH10B, DP50supF, ED8654, ED8767, ER1647, HB101, HMS174, HST02, HST04dam⁻/dcm⁻, HST08Premium, JM83, JM101, JM105, JM106, JM107, JM108, JM109, JM110, K802, K803, LE392, MC1061, MV1184, MV1193, NovaBlue, RR1, TAP90, TG1, TG2, TH2, XL1-Blue, X-1776, γ-1088, γ-1089, and γ-1090.

The introduction of the vector into the host cell (transformation) can be carried out by a conventionally known method, such as the electroporation method and the calcium method, according to the host cell to be used. In addition, extraction of the mutated bilirubin oxidase from the transformant cultured and purification of the extract can be carried out, for example, by breaking up the cells by ultrasonic pulverization, and removing unpulverized cells and precipitates from the pulverization product by centrifugation, followed by dialysis. The crude product thus obtained may be subjected further to purification by liquid chromatography.

### Examples

### <Experimental Example 1>

1. cDNA cloning of bilirubin oxidase derived from imperfect filamentous fungus Myrothecium verrucaria NBRC (IFO) 6113 strain

### (1-1) Culture of Myrothecium Verrucaria

Myrothecium verrucaria NBRC (IFO) 6113 strain was purchased from the independent administrative agency National Institute of Technology and Evaluation (NITE), Biotechnology Center, in lots. The purchased dried fungal body was suspended in a rehydration fluid (polypeptone: 0.5%, yeast extract: 0.3%, MgSO₄·7H₂O: 0.1%), and the resulting suspension was inoculated into a potato dextrose agar (PDA) plate (potato dextrode: 2.4%, agarose: 1.5%). Upon culture at room temperature for five to seven days, the surfaces of the PDA plate were covered with white hyphae. The hyphae were scraped by a spatula, and stored at -80°C. The yield of the cell body was 50 to 60 mg (wet mass) per PDA plate (diameter: 9 cm).

### (1-2) RNA extraction and cDNA synthesis

From about 100 mg of frozen fungal body powder of Myrothecium verrucaria NBRC (IFO) 6113 strain, 100 µm (determined by UV absorption) of total RNA was extracted. The total RNA was used as a template RNA for reverse transcription PCR.

The reverse transcription PCR was performed using the total RNA as a template and using OneStep RT-PCR Kit (produced by Qiagen K.K.). The base sequence of the primer used for the reverse transcription PCR was designed as shown in Table 1 below, referring to the base sequence of cDNA of BO which had been reported.

**[Table 1]**

| Primer | Base sequence | SEQ ID No. |
|---|---|---|
| Forward primer | 5′- GATAT ACATA TGGTT GCCCA GATCA GCCCA CAGTA TC -3′ | 3 |
| Reverse primer | 5′- GCTCG AATTC TTACT CGTCA GCTGC GGCGT AAGG -3′ | 4 |

(In the base sequence, the underlined parts represent restriction enzyme sites, which are an NdeI site (CATATG) and an EcoRI site (GAATTC), respectively.)

As a result of agarose gel electrophoresis of the PCR product, an amplified fragment containing the desired bilirubin oxidase gene was confirmed to be present in the vicinity of 1600 bp. This amplified fragment was cut out from the agarose gel slab, and used in the subsequent step.

### (1-3) Cloning

The amplified fragment was digested by restriction enzymes NdeI and EcoRI, after which the digestion product was put to a ligation reaction with pET22b+ plasmid vector digested by the same enzymes. The plasmid vector after the ligation was introduced into Escherichia coli TOP10 strain (produced by Invitrogen Corporation), followed by inoculation onto an LB/Amp agar plate medium. After culturing overnight, a colony of a transformant having drug resistance to ampicillin was obtained. The colony was put to subculture on 3 ml of an LB/Amp medium overnight, after which a plasmid vector was isolated from the resulting cell body.

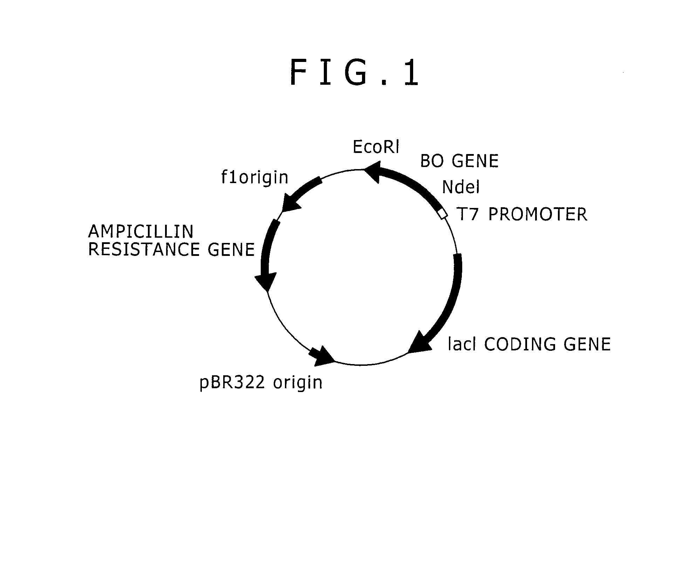

Upon examination of the base sequence of the insertion region of the plasmid vector, it was found to be the base sequence of SEQ ID NO: 2. The base sequence of SEQ ID NO: 2 is 1608 bp (inclusive of ATG as an initiation codon and TAA as a termination codon), which corresponds to 535 amino acid residues (inclusive of methionine derived from the initiation codon) described in SEQ ID NO: 1. The amino acid sequence coincided with the amino acid sequence of a mature enzyme of the natural bilirubin oxidase derived from Myrothecium verrucaria. Hereafter, this plasmid vector will be referred to as "pET-BO vector." FIG. 1 shows the structure of the pET-BO vector.

### <Experimental Example 2>

### 2. Expression of recombinant bilirubin oxidase in Escherichia coli

### (2-1) Transformation of Escherichia coli and expression of recombinant bilirubin oxidase

Using the above-mentioned pET-BO vector, transformation of Escherichia coli (BL21 (DE3) strain derived from K-12) was carried out. The colony of the transformant thus obtained was inoculated into an LB/Amp liquid medium, and subjected to shake culture at 37°C for four hours. Then, 1 ml of the cell body liquid thus obtained was put to subculture on 100 mL of a TB/Amp medium, and subjected to shake culture at 37°C for four hours.

After adding IPTG (final concentration: 0.25 mM) and copper sulfate pentahydrate (2.0 mM) to the medium, culture was performed at 18°C for 16 hours. After the culture, the resulting cell body was recovered by centrifugation. The cell body was resuspended in 50 mM of Tris-hydrochloric acid buffer (pH: 7.6, containing 0.1 mM of PMSF and 0.3 mM of copper sulfate pentahydrate), and was pulverized by ultrasonic pulverization. After unpulverized cells and precipitates were removed from the pulverization product by centrifugation, the resulting product was dialyzed with respect to 50 mM of the Tris-hydrochloric acid buffer (pH 7.6).

### (2-2) Purification of recombinant bilirubin oxidase by anion exchange column

Purification of the recombinant bilirubin oxidase was carried out by anion exchange chromatography based on a DEAE cellulose resin (Hi-trap DEAE (5 ml), produced by GE Healthcare). The dialyzed sample was adsorbed on the column, and the recombinant bilirubin oxidase thus adsorbed was eluted with linear gradient (0-50%) using 50 mM of Tris-hydrochloric acid buffer (pH 7.6) containing 50 mM of NaCl.

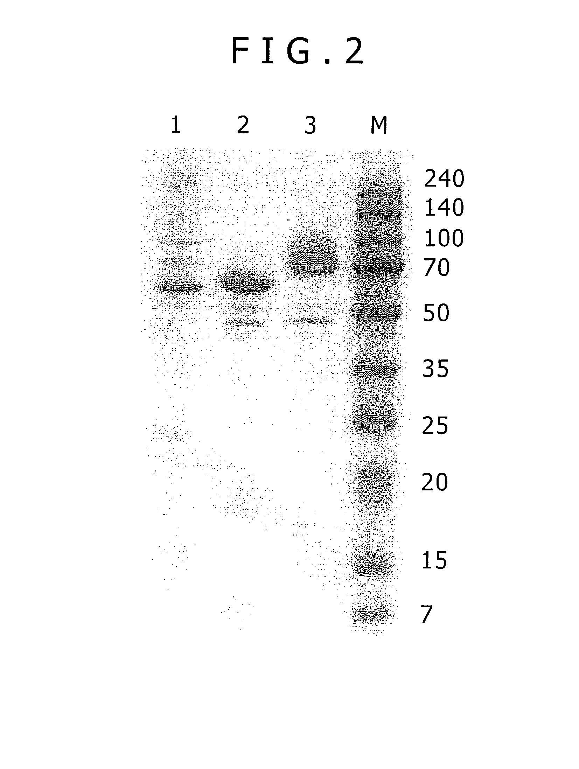

The SDS-PAGE of the recombinant bilirubin oxidase after the purification was repeated twice by the above-mentioned method is shown in FIG. 2. In the figure, lane 1 corresponds to the purified recombinant bilirubin oxidase, lane 2 corresponds to a commercially available natural bilirubin oxidase (BO) ("Amono" 3 (BO-3), produced by Amano Enzyme Inc.), and lane 3 corresponds to recombinant bilirubin oxidase obtained by expression in yeast. In addition, lane M is a size marker.

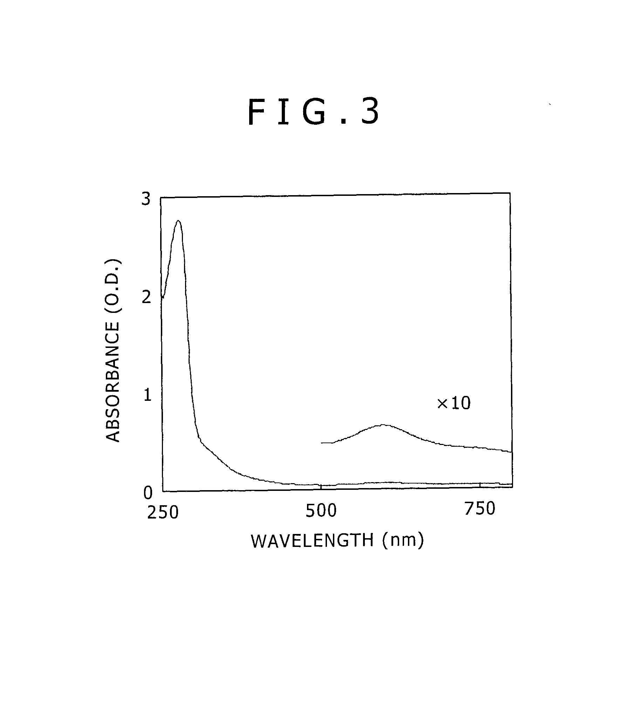

Besides, absorption spectrum of the purified recombinant bilirubin oxidase is shown in FIG. 3. The yield of the BO per 100 mL of the medium, estimated from the absorbance difference (Δε = 4000) between 600 nm and 500 nm in the absorption spectrum, was 0.6 mg.

### <Experimental Example 3>

### 3. Measurement of activity of recombinant bilirubin oxidase

The activity of the recombinant bilirubin oxidase was evaluated, and compared with that of a commercially available natural bilirubin oxidase. The activity measurement was conducted using ABTS as a substrate, and variation in absorbance at 730 nm attendant on the progress of reaction (which arises from an increase in the amount of reaction product of ABTS) was traced. The measurement conditions are set forth in Table 2. Incidentally, the concentration of the bilirubin oxidase was so prepared that the variation in absorbance in 730 nm would be about 0.01 to 0.5 per minute. The reaction was initiated by addition of the enzyme solution (5 µL) to a phosphate buffer (2995 µL) containing 0.05 to 10 mM of ABTS.

**[Table 2]**

| | |
|---|---|
| Buffer | 46.5 mM aqueous Na phosphate solution (pH 7.0) |
| ABTS concentration | 0.05 to 10 mM (final concentration) |
| O₂ concentration | saturated (210 µM; 25°C) |
| Reaction temperature | 25°C |

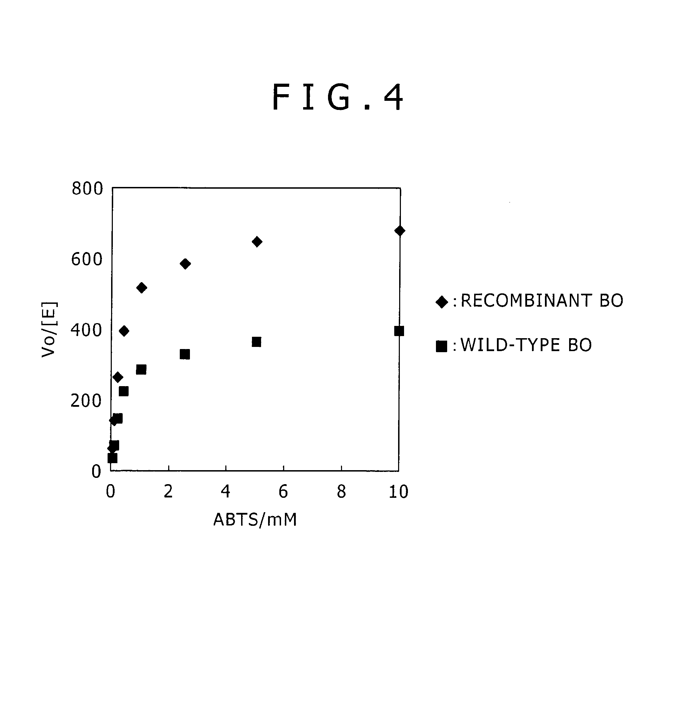

The results of the activity evaluation are shown in FIG. 4. In addition, Kₘ and k_{cat} values of the recombinant bilirubin oxidase and the commercially available natural bilirubin oxidase are set forth in Table 3. The recombinant bilirubin oxidase obtained using Escherichia coli showed an activity (k_{cat}) of about 1.8 times that of the natural bilirubin oxidase, whereby its excellent activity was verified.

**[Table 3]**

| | Kₘ/mM | K_{cat}/s⁻¹ |
|---|---|---|
| Recombinant BO | 0.34 ± 0.02 | 690 ± 52 |
| Wild-type BO | 0.38 ± 0.05 | 393 ± 11 |

### Industrial Applicability

By the method of producing a recombinant bilirubin oxidase according to the present invention, a bilirubin oxidase derived from imperfect filamentous fungus Myrothecium verrucaria can be provided in large amounts through a very easy and economical method. Furthermore, the recombinant bilirubin oxidase thus obtained has a high enzymatic activity equivalent to or higher than that of the natural bilirubin oxidase. Accordingly, the present invention is applicable to inexpensive supply of a bilirubin oxidase derived from imperfect filamentous fungus Myrothecium verrucaria, as a reagent for clinical tests, an electrode enzyme for enzyme cells, or the like.

## Claims

1. A recombinant bilirubin oxidase obtained by introducing bilirubin oxidase gene derived from imperfect filamentous fungus Myrothecium verrucaria to Escherichia coli and allowing expression thereof.

2. The recombinant bilirubin oxidase according to claim 1, having an enzymatic activity higher than that of a natural bilirubin oxidase derived from the imperfect filamentous fungus Myrothecium verrucaria.

3. The recombinant bilirubin oxidase according to claim 2, having the amino acid sequence of SEQ ID NO: 1.

4. The recombinant bilirubin oxidase according to any one of claims 1 to 3, used as an electrode enzyme of an enzyme cell.

5. A method of preparing a recombinant bilirubin oxidase, comprising a step wherein a bilirubin oxidase gene derived from imperfect filamentous fungus Myrothecium verrucaria is introduced to Escherichia coli and expressed.

6. The method according to claim 5, wherein an Escherichia coli strain derived from K-12 is used.

7. The method according to claim 5 or 6, wherein a bilirubin oxidase gene derived from imperfect filamentous fungus Myrothecium verrucaria which has the base sequence of SEQ ID NO: 2 is used.


SEQUENCE LISTING

<110>  Sony Corporation

<120>  RECOMBINANT BILIRUBIN OXIDASE AND METHOD FOR PRODUCING THE SAME

<130>  S11P0836WO00

<150> JP 2010-111186
<151> 2010-05-13

<160>  4

<170>  PatentIn version 3.5

<210>  1
<211>  535
<212>  PRT
<213>  Myrothecium verrucaria

<400>  1

Met Val Ala Gln Ile Ser Pro Gln Tyr Pro Met Phe Thr Val Pro Leu
1               5                   10                  15

Pro Ile Pro Pro Val Lys Gln Pro Arg Leu Thr Val Thr Asn Pro Val
            20                  25                  30

Asn Gly Gln Glu Ile Trp Tyr Tyr Glu Val Glu Ile Lys Pro Phe Thr
        35                  40                  45

His Gln Val Tyr Pro Asp Leu Gly Ser Ala Asp Leu Val Gly Tyr Asp
    50                  55                  60

Gly Met Ser Pro Gly Pro Thr Phe Gln Val Pro Arg Gly Val Glu Thr
65                  70                  75                  80

Val Val Arg Phe Ile Asn Asn Ala Glu Ala Pro Asn Ser Val His Leu
                85                  90                  95

His Gly Ser Phe Ser Arg Ala Ala Phe Asp Gly Trp Ala Glu Asp Ile
            100                 105                 110

Thr Glu Pro Gly Ser Phe Lys Asp Tyr Tyr Tyr Pro Asn Arg Gln Ser
        115                 120                 125

Ala Arg Thr Leu Trp Tyr His Asp His Ala Met His Ile Thr Ala Glu
    130                 135                 140

Asn Ala Tyr Arg Gly Gln Ala Gly Leu Tyr Met Leu Thr Asp Pro Ala
145                 150                 155                 160

Glu Asp Ala Leu Asn Leu Pro Ser Gly Tyr Gly Glu Phe Asp Ile Pro
                165                 170                 175

Met Ile Leu Thr Ser Lys Gln Tyr Thr Ala Asn Gly Asn Leu Val Thr
            180                 185                 190

Thr Asn Gly Glu Leu Asn Ser Phe Trp Gly Asp Val Ile His Val Asn
        195                 200                 205

Gly Gln Pro Trp Pro Phe Lys Asn Val Glu Pro Arg Lys Tyr Arg Phe
    210                 215                 220

Arg Phe Leu Asp Ala Ala Val Ser Arg Ser Phe Gly Leu Tyr Phe Ala
225                 230                 235                 240

Asp Thr Asp Ala Ile Asp Thr Arg Leu Pro Phe Lys Val Ile Ala Ser
                245                 250                 255

Asp Ser Gly Leu Leu Glu His Pro Ala Asp Thr Ser Leu Leu Tyr Ile
            260                 265                 270

Ser Met Ala Glu Arg Tyr Glu Val Val Phe Asp Phe Ser Asp Tyr Ala
        275                 280                 285

Gly Lys Thr Ile Glu Leu Arg Asn Leu Gly Gly Ser Ile Gly Gly Ile
    290                 295                 300

Gly Thr Asp Thr Asp Tyr Asp Asn Thr Asp Lys Val Met Arg Phe Val
305                 310                 315                 320

Val Ala Asp Asp Thr Thr Gln Pro Asp Thr Ser Val Val Pro Ala Asn
                325                 330                 335

Leu Arg Asp Val Pro Phe Pro Ser Pro Thr Thr Asn Thr Pro Arg Gln
            340                 345                 350

Phe Arg Phe Gly Arg Thr Gly Pro Thr Trp Thr Ile Asn Gly Val Ala
        355                 360                 365

Phe Ala Asp Val Gln Asn Arg Leu Leu Ala Asn Val Pro Val Gly Thr
    370                 375                 380

Val Glu Arg Trp Glu Leu Ile Asn Ala Gly Asn Gly Trp Thr His Pro
385                 390                 395                 400

Ile His Ile His Leu Val Asp Phe Lys Val Ile Ser Arg Thr Ser Gly
                405                 410                 415

Asn Asn Ala Arg Thr Val Met Pro Tyr Glu Ser Gly Leu Lys Asp Val
            420                 425                 430

Val Trp Leu Gly Arg Arg Glu Thr Val Val Val Glu Ala His Tyr Ala
      435                 440                 445

Pro Phe Pro Gly Val Tyr Met Phe His Cys His Asn Leu Ile His Glu
    450                 455                 460

Asp His Asp Met Met Ala Ala Phe Asn Ala Thr Val Leu Pro Asp Tyr
465                 470                 475                 480

Gly Tyr Asn Ala Thr Val Phe Val Asp Pro Met Glu Glu Leu Trp Gln
                485                 490                 495

Ala Arg Pro Tyr Glu Leu Gly Glu Phe Gln Ala Gln Ser Gly Gln Phe
            500                 505                 510

Ser Val Gln Ala Val Thr Glu Arg Ile Gln Thr Met Ala Glu Tyr Arg
      515                 520                 525

Pro Tyr Ala Ala Ala Asp Glu
    530                 535

<210>  2
<211>  1608
<212>  DNA
<213>  Myrothecium verrucaria

<400>  2
atggttgccc agatcagccc acagtatccc atgttcacag taccactgcc aattcctcct      60

gttaagcagc cccgcttgac tgtaaccaat cctgtgaatg gacaagagat ctggtactat     120

gaggtcgaga tcaagccctt cactcaccag gtttaccctg accttggatc cgctgatctg     180

gtcgggtacg atggaatgtc tcctggccct actttccagg ttcctcgtgg agttgaaaca     240

gttgtccgct tcattaacaa tgctgaggct cctaactctg ttcacttgca cggatcattc     300

tctcgtgccg cctttgacgg atgggcagag gacatcaccg agcctggcag cttcaaagac     360

tattactacc caaacagaca gtctgcccgt accctatggt accacgatca tgctatgcat     420

atcactgctg agaacgccta ccgtggccag gctggtctct acatgctcac tgacccagcc     480

gaagacgctc tcaacttgcc aagtggatat ggcgagttcg atattccaat gatcctcacg     540

tctaagcaat acaccgcaaa cggcaacttg gtcaccacta atggagagct gaactcattc     600

tggggtgatg taattcacgt gaacggtcaa ccctggcctt tcaagaacgt tgagcctcgc     660

aaatatcgat tccgcttcct cgatgccgca gtttctcgct ctttcggcct ttactttgct     720

gatactgatg ctatcgacac tcgcttgcct ttcaaggtta ttgcctccga ttctggtctt     780

cttgaacacc ctgccgatac cagcttgctg tacatttcca tggccgagcg ttacgaagtt     840

gtgtttgact tctccgacta tgctggcaag actattgaac tccgcaacct gggcggcagc     900

attggcggca ttggaacaga taccgactat gataacaccg acaaggtcat gcgtttcgtg     960

gtagcagacg acacaactca gccagatacc tcagttgttc ctgctaacct tcgtgatgtt 1020

cccttcccct ctcccaccac aaacaccccc cgacagttcc gctttggtcg caccggtcct 1080

acctggacta ttaatggtgt tgcttttgct gatgttcaaa accgtctgct tgcaaacgta 1140

cccgttggta ctgtcgagcg ttgggagctc atcaacgccg gtaacggttg gacgcaccct 1200

attcacatcc atcttgtcga cttcaaggtc atttctcgta cttccggcaa caacgcgcgc 1260

acagtcatgc catacgagtc cggtctcaaa gacgttgtct ggcttggtcg ccgtgaaact 1320

gtggttgttg aggctcatta cgcgcctttc cctggtgtat acatgttcca ttgccacaat 1380

ttgattcacg aggatcacga tatgatggct gcctttaacg ccaccgtcct gccagattat 1440

ggctataatg ccactgtttt cgttgaccct atggaagagc tttggcaggc tcgtccctat 1500

gaactcggcg agttccaggc tcagagtggc cagttcagcg ttcaggctgt tactgagcgt 1560

atccagacta tggctgaata cagaccttac gccgcagctg acgagtag 1608

<210> 3
<211> 37
<212> DNA
<213> Artificial Sequence

<220>
<223> Forward pimer for RT-PCR

<400> 3
gatatacata tggttgccca gatcagccca cagtatc 37

<210> 4
<211> 34
<212> DNA
<213> Artificial Sequence

<220>
<223> Reverse primer for RT-PCR

<400> 4
gctcgaattc ttactcgtca gctgcggcgt aagg 34